(19) Europäisches Patentamt
European Patent Office
Office européen des brevets

(11) Veröffentlichungsnummer: **0 123 998**
**A2**

(12) **EUROPÄISCHE PATENTANMELDUNG**

(21) Anmeldenummer: **84104256.7**

(22) Anmeldetag: **14.04.84**

(51) Int. Cl.³: **C 07 D 491/048**, A 61 K 31/435
//
(C07D491/048, 307/00, 221/00)

(30) Priorität: **27.04.83 DE 3315157**

(43) Veröffentlichungstag der Anmeldung: **07.11.84**
**Patentblatt 84/45**

(84) Benannte Vertragsstaaten: **AT BE CH DE FR IT LI LU NL SE**

(71) Anmelder: **BOEHRINGER INGELHEIM KG,**
**D-6507 Ingelheim am Rhein (DE)**

(72) Erfinder: **Schneider, Claus, Dr.,**
**Albrecht-Dürer-Strasse 19, D-6507 Ingelheim/Rhein (DE)**
Erfinder: **Walther, Gerhard, Dr.,**
**Pfarrer-Heberer-Strasse 37, D-6530 Bingen/Rhein (DE)**
Erfinder: **Weber, Karl-Heinz, Dr., Kaiser-Karl-Strasse 11,**
**D-6535 Gau-Algesheim (DE)**
Erfinder: **Bechtel, Wolf Dietrich, Dr., Mühlstrasse 3,**
**D-6531 Appenheim (DE)**
Erfinder: **Böke-Kuhn, Karin, Dr., Beethovenstrasse 11,**
**D-6535 Gau-Algesheim (DE)**

(54) **4-Phenyl-tetrahydro-furano-pyridine, Verfahren zu ihrer Herstellung und pharmazeutische Zusammensetzungen.**

(57) Die Erfindung betrifft neue 4-Phenyl-tetrahydro-furano-pyridine der allgemeinen Formeln

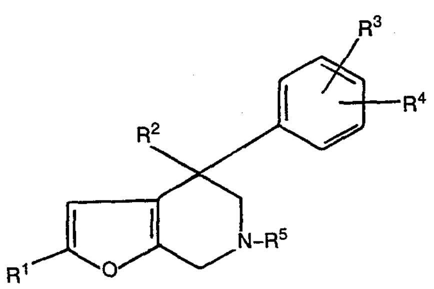

(I)

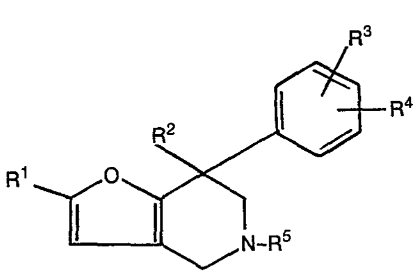

(II)

worin
$R^1$ Wasserstoff, Fluor, Chlor, Brom, eine Alkylgruppe mit 1–4 Kohlenstoffatomen, eine Hydroxyalkylgruppe mit 1–2 Kohlenstoffatomen, eine Alkoxyalkylgruppe, wobei jeder Alkylrest 1–2 Kohlenstoffatome enthalten kann, die Pyrrolidinomethylgruppe oder eine Dialkylaminomethylgruppe, wobei jeder Alkylrest 1–2 Kohlenstoffatome enthalten kann;
$R^2$ Wasserstoff, Hydroxy, Methoxy oder Ethoxy;
$R^3$ und $R^4$, die gleich oder verschieden sein können, Fluor, Chlor, Brom, Methyl, Ethyl, Hydroxy, Methoxy, Acetoxy, Benzyloxy oder
$R^3$ und $R^4$ bilden zusammen mit dem Phenylring eine 3,4-Benzodioxanylgruppe;
$R^5$ Wasserstoff, einen Alkylrest mit 1–4 Kohlenstoffatomen, den Benzyl- oder o-Chlorbenzylrest bedeuten mit der Maßgabe, daß in Formel II $R^2$ Wasserstoff und $R^1$ nicht Hydroxyalkyl bedeutet, ihre physiologisch verträglichen Säureadditionssalze sowie Verfahren zur Herstellung der neuen Verbindungen und ihre Verwendung in Arzneimitteln.

Die neuen Verbindungen sollen als Antidepressiva mit insbesondere thymoleptischen und zentralanregenden Eigenschaften eingesetzt werden.

EP 0 123 998 A2

0123998

Die Erfindung betrifft neue 4-Phenyl-tetrahydro-furano-pyridine, Verfahren zu ihrer Herstellung und diese enthaltende pharmazeutische Zusammensetzungen.

In den neuen Verbindungen der allgemeinen Formeln

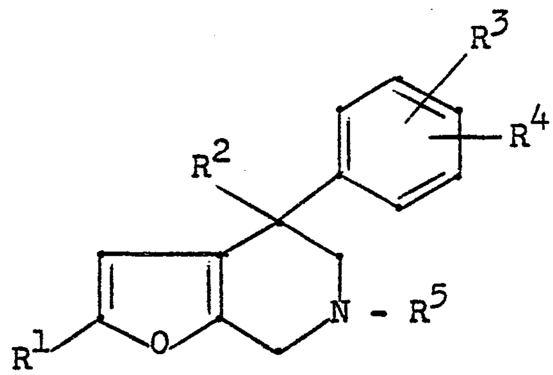

(I)

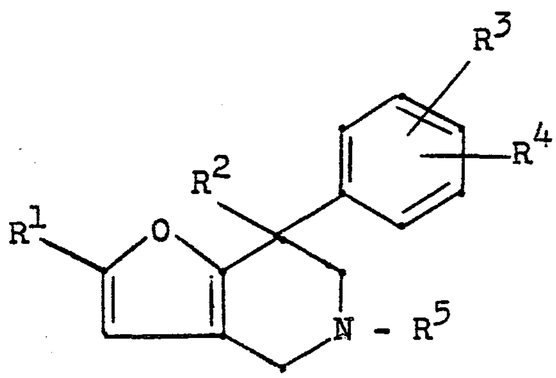

(II)

bedeuten:

$R^1$ Wasserstoff, Fluor, Chlor, Brom, eine Alkylgruppe mit 1 - 4 Kohlenstoffatomen, eine Hydroxyalkylgruppe mit 1 - 2 Kohlenstoffatomen, eine Alkoxyalkylgruppe, wobei jeder Alkylrest 1 - 2 Kohlenstoffatome enthalten kann, die Pyrrolidinomethylgruppe oder eine Dialkylaminomethylgruppe, wobei jeder Alkylrest 1 - 2 Kohlenstoffatome enthalten kann;

$R^2$ Wasserstoff, Hydroxy, Methoxy oder Ethoxy;

$R^3$ und $R^4$, die gleich oder verschieden sein können, Fluor, Chlor, Brom, Methyl, Ethyl, Hydroxy, Methoxy, Acetoxy, Benzyloxy oder

$R^3$ und $R^4$ bilden zusammen mit dem Phenylring eine 3,4-Benzodioxanylgruppe;

$R^5$ Wasserstoff, einen Alkylrest mit 1 - 4 Kohlenstoffatomen, den Benzyl- oder o-Chlorbenzylrest, mit der Maßgabe, daß in Formel II $R^2$ Wasserstoff und $R^1$ nicht Hydroxyalkyl bedeutet.

Die Erfindung umfaßt ferner die physiologisch verträglichen Säureadditionssalze der vorstehend genannten Endprodukte.

Die neuen Verbindungen können nach verschiedenen Verfahren hergestellt werden.
Verbindungen der allgemeinen Formel I erhält man beispielsweise durch Umsetzung eines 4,5,6,7-Tetrahydro-4-hydroxy-furano[2,3-c]pyridins der allgemeinen Formel

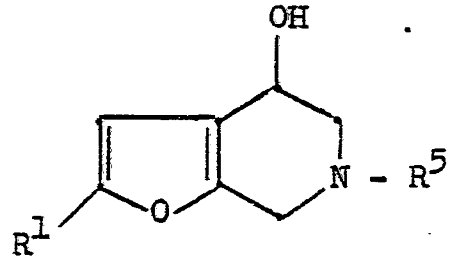

(III)

worin
$R^1$ und $R^5$ die oben angegebene Bedeutung haben, mit einem Benzolderivat der allgemeinen Formel

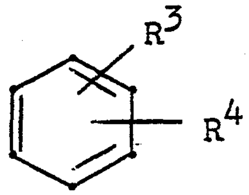

(IV)

worin
$R^3$ und $R^4$ die oben angeführte Bedeutung haben.

Die Umsetzung erfolgt, je nach Art des eingesetzten Benzolderivates, unter Verwendung saurer Katalysatoren, wie Methansulfonsäure oder Aluminiumchlorid, bei Raumtemperatur oder unter Kühlung. Enthält eine Verbindung der allgemeinen Formel IV nur einen Substituenten, so erhält man bei dieser Reaktion sowohl das o- als auch das p-Isomere, die chromatographisch getrennt werden können.
Nach diesem Verfahren erhält man solche Verbindungen der allgemeinen Formel I, in denen $R^2$ Wasserstoff bedeutet.

Zur Herstellung solcher Endprodukte der allgemeinen Formel I, in denen $R^2$ die Hydroxygruppe bedeutet, führt man eine Verbindung der allgemeinen Formel III mittels eines Ketons, wie Aceton oder Cyclohexanon, und eines Aluminiumalkoxylats unter den Bedingungen der Oppenauer-Oxidation in die entsprechende 4-Oxo-Verbindung über. Hierbei wird das Ausgangsprodukt der Formel III zweckmäßigerweise in einem inerten organischen Lösungsmittel, wie Toluol, Benzol, Xylol usw. gelöst und unter Zusatz des Ketons und des Aluminiumalkoxylats unter Rückfluß

erhitzt. Die so erhaltene 4-Oxo-Verbindung der allgemeinen Formel

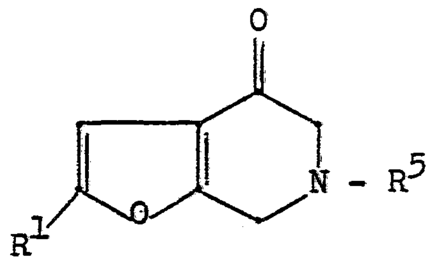

(V)

worin
$R^1$ und $R^5$ die oben angeführte Bedeutung haben, wird sodann mit einer Lösung eines entsprechenden Phenylmagnesiumbromids der allgemeinen Formel

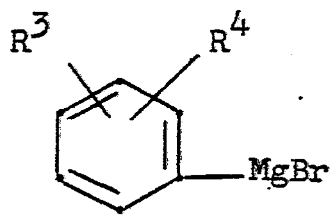

(VI)

worin
$R^3$ und $R^4$ die vorstehend angegebene Bedeutung haben, unter Kühlung zur Reaktion gebracht.

Verbindungen der allgemeinen Formel II erhält man, ausgehend von einem entsprechenden 2-Furfurylamino-1-phenyl-ethanol der allgemeinen Formel

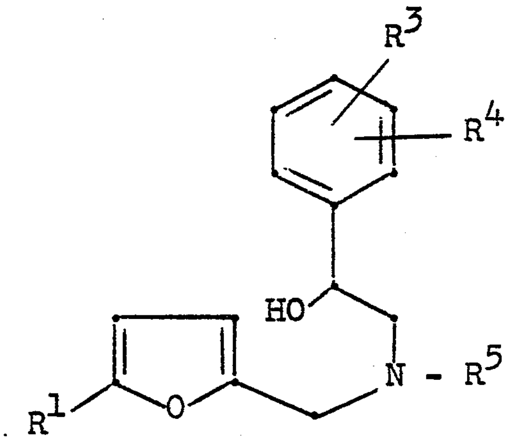

(VII)

worin
die Reste $R^1$, $R^3$, $R^4$ und $R^5$ die oben angeführte Bedeutung für Verbindungen der allgemeinen Formel II haben, durch Kondensation mit Trifluoressigsäure oder Trifluorsulfonsäure. Hierbei wird das Ausgangsprodukt VII in einem inerten organischen Lösungsmittel, z.B. Ethylenchlorid, gelöst und mit der Säure unter Rückfluß erhitzt.

Steht $R^5$ für den Benzyl- oder den o-Chlorbenzylrest, so läßt sich ein solches Endprodukt nach üblichen Methoden, vorzugsweise katalytisch, mit Palladium/Kohle, entbenzylieren.
Es ist ebenfalls möglich, in ein Endprodukt der allgemeinen Formel I, in dem $R^1$ Wasserstoff bedeutet, ein Halogenatom oder eine Dialkylaminogruppe beziehungsweise eine Pyrrolidinomethylgruppe einzuführen. Zu diesem Zweck wird die Verbindung mit $R^1$ = Wasserstoff in einem inerten organischen Lösungsmittel, z.B. Acetonitril, gelöst und einige Zeit mit Dialkylamin und Formaldehyd beziehungsweise einem Halogenierungsmittel erhitzt.

Aus einer Verbindung der allgemeinen Formel I, in der $R^2$ die Hydroxygruppe bedeutet, kann diese Gruppe auf folgendem Weg abgespalten werden:

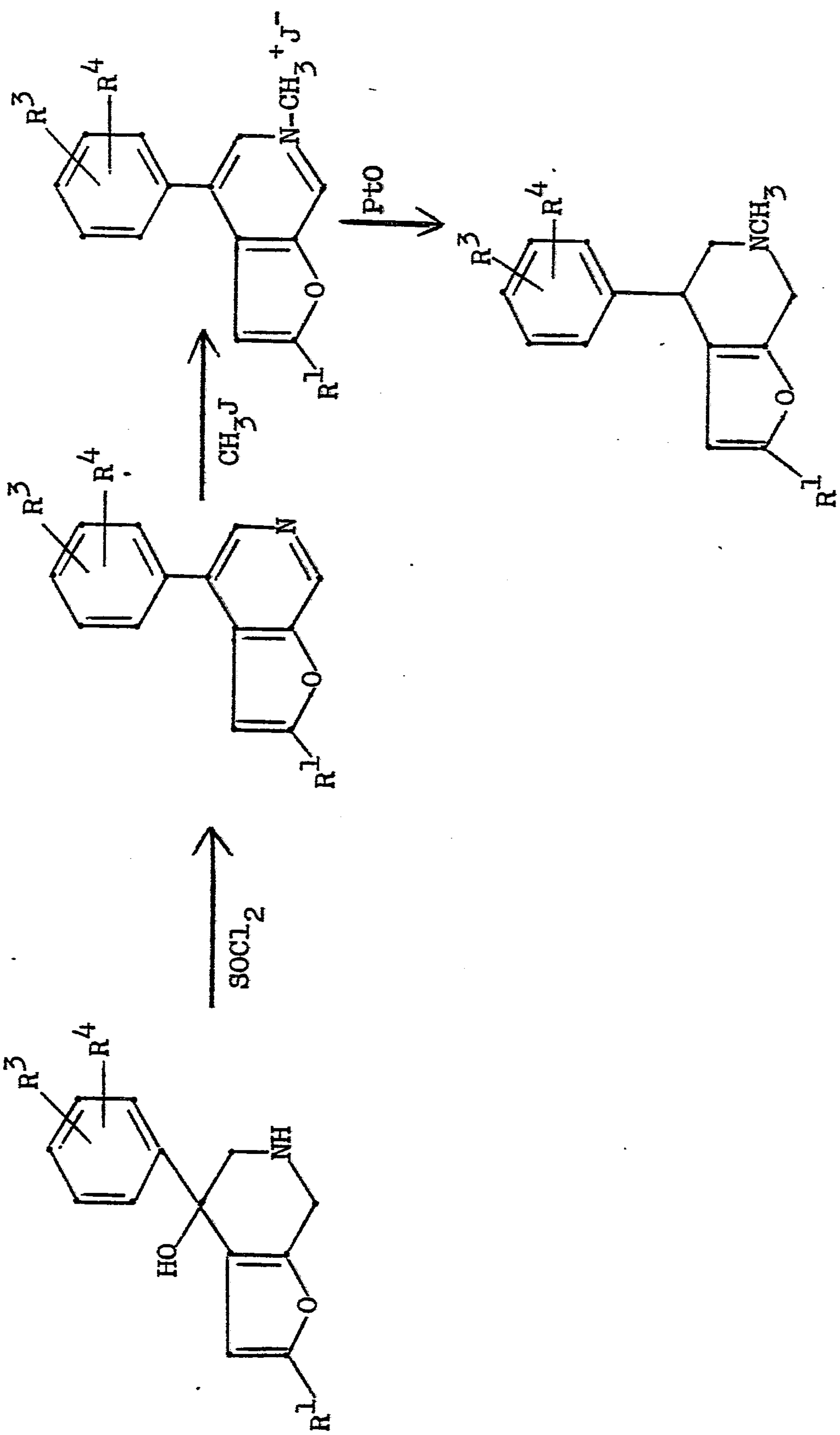
SOCl$_2$
CH$_3$J
PtO

Zur Herstellung von Endprodukten mit $R^1$ = Hydroxyalkyl setzt man zweckmäßigerweise bereits eine Ausgangsverbindung III oder IV ein, die in 2-Stellung eine Alkoxycarbonylgruppe aufweist. Nach Ankondensieren des Benzolringes in 4-Stellung erfolgt dann gewünschtenfalls die Umwandlung der Alkoxycarbonylgruppe in die entsprechende Hydroxyalkylgruppe, vorzugsweise mit Lithiumalanat in einem inerten organischen Lösungsmittel, wie Tetrahydrofuran, bei Raumtemperatur.

Bedeutet einer oder beide der Reste $R^3$ und $R^4$ eine Hydroxygruppe, so können diese auf bekannte Weise, z.B. durch Umsetzung mit Bortrifluorid/Methanol in eine Methoxygruppe oder, z.B. durch Erhitzen mit Acetanhydrid, in eine Acetoxygruppe umgewandelt werden.

Nach den oben beschriebenen Verfahren können beispielsweise die folgenden Endprodukte, gegebenenfalls in Form ihrer Säureadditionssalze, erhalten werden:

2,6-Dimethyl-4-hydroxy-4-(p-bromphenyl)-4,5,6,7-tetrahydro-furano[2,3-c]pyridin,

4-Hydroxy-4-(p-bromphenyl)-6-methyl-4,5,6,7-tetrahydro-furano[2,3-c]pyridin,

4-Methoxy-4-(p-bromphenyl)-6-methyl-4,5,6,7-tetrahydro-furano[2,3-c]pyridin,

2-Hydroxymethyl-4-hydroxy-4-(p-tolyl)-4,5,6,7-tetrahydro-furano[2,3-c]pyridin,

2,6-Dimethyl-4-hydroxy-4-(m-methoxyphenyl)-4,5,6,7-tetrahydro-furano[2,3-c]pyridin,

2,6-Dimethyl-4-hydroxy-4-(p-tolyl)-4,5,6,7-tetrahydro-furano[2,3-c]pyridin,

2,6-Dimethyl-4-hydroxy-4-(p-ethylphenyl)-4,5,6,7-tetrahydro-furano[2,3-c]pyridin,

2,6-Dimethyl-4-hydroxy-4-(p-chlorphenyl)-4,5,6,7-tetrahydro-furano[2,3-c]pyridin,

4-(p-Tolyl)-6-methyl-4,5,6,7-tetrahydro-furano[2,3-c]-pyridin,

2,6-Dimethyl-4-(3,4-dihydroxyphenyl)-4,5,6,7-tetrahydro-furano[2,3-c]pyridin,

2-Hydroxymethyl-4-(p-tolyl)-6-methyl-4,5,6,7-tetrahydro-furano[2,3-c]pyridin,

4-(p-Hydroxyphenyl)-6-methyl-4,5,6,7-tetrahydro-furano-[2,3-c]pyridin,

4-(o-Hydroxyphenyl)-4,5,6,7-tetrahydro-furano[2,3-c]-pyridin,

2,6-Dimethyl-4-(p-hydroxyphenyl)-4,5,6,7-tetrahydro-furano[2,3-c]pyridin,

2,6-Dimethyl-4-(o-hydroxyphenyl)-4,5,6,7-tetrahydro-furano[2,3-c]pyridin,

2,6-Dimethyl-4-(p-acetoxyphenyl)-4,5,6,7-tetrahydro-furano[2,3-c]pyridin,

2,6-Dimethyl-4-(p-bromphenyl)-4,5,6,7-tetrahydro-furano-[2,3-c]pyridin,

2,6-Dimethyl-4-(o-bromphenyl)-4,5,6,7-tetrahydro-furano[2,3-c]pyridin,

2-Methyl-4-(p-methoxyphenyl)-4,5,6,7-tetrahydro-furano-[2,3-c]pyridin,

4-(3,4-Dihydroxyphenyl)-6-methyl-4,5,6,7-tetrahydro-furano[2,3-c]pyridin,

4-(p-Methoxyphenyl)-6-methyl-4,5,6,7-tetrahydro-furano[2,3-c]pyridin,

4-(o-Methoxyphenyl)-6-methyl-4,5,6,7-tetrahydro-furano[2,3-c]pyridin,

2,6-Dimethyl-4-(p-chlorphenyl)-4,5,6,7-tetrahydro-furano[2,3-c]pyridin,

2,6-Dimethyl-4-(o-chlorphenyl)-4,5,6,7-tetrahydro-furano[2,3-c]pyridin,

2,6-Dimethyl-4-(p-fluorphenyl)-4,5,6,7-tetrahydro-furano[2,3-c]pyridin,

2,6-Dimethyl-4-(o-fluorphenyl)-4,5,6,7-tetrahydro-furano[2,3-c]pyridin,

2-Methyl-4-(p-bromphenyl)-4,5,6,7-tetrahydro-furano-[2,3-c]pyridin,

2-Methyl-4-(o-bromphenyl)-4,5,6,7-tetrahydro-furano-[2,3-c]pyridin,

2-Methyl-4-phenyl-4,5,6,7-tetrahydro-furano[2,3-c]-pyridin,

2-Pyrrolidinomethyl-4-(p-methoxyphenyl)-6-methyl-4,5,6,7-tetrahydro-furano[2,3-c]pyridin,

2-Methyl-4-phenyl-6-ethyl-4,5,6,7-tetrahydro-furano-[2,3-c]pyridin,

2-Methyl-4-phenyl-6-n-propyl-4,5,6,7-tetrahydro-furano[2,3-c]pyridin,

4-(p-Fluorphenyl)-4,5,6,7-tetrahydro-furano[2,3-c]-pyridin,

4-(3,4-Dihydroxyphenyl)-4,5,6,7-tetrahydro-furano-[2,3-c]pyridin,

2-Methyl-4-(3,4-dihydroxyphenyl)-4,5,6,7-tetrahydro-furano[2,3-c]pyridin,

2-Methyl-4-phenyl-6-n-butyl-4,5,6,7-tetrahydro-furano-[2,3-c]pyridin,

2-Methyl-4-(1,4-benzodioxanyl)-4,5,6,7-tetrahydro-furano[2,3-c]pyridin,

4-Phenyl-6-methyl-4,5,6,7-tetrahydro-furano[2,3-c]-pyridin,

2-Ethyl-4-(3,4-dihydroxyphenyl)-6-methyl-4,5,6,7-tetrahydro-furano[2,3-c]pyridin.

Ferner wurden folgende Endprodukte der allgemeinen Formel II erhalten:

2,6-Dimethyl-4-phenyl-4,5,6,7-tetrahydro-furano[3,2-c]-pyridin,

4-Phenyl-4,5,6,7-tetrahydro-furano[3,2-c]pyridin,

4-(p-Tolyl)-6-methyl-4,5,6,7-tetrahydro-furano[3,2-c]-pyridin,

4-Phenyl-6-methyl-4,5,6,7-tetrahydro-furano[3,2-c]-pyridin,

4-(p-Bromphenyl)-6-methyl-4,5,6,7-tetrahydro-furano-[3,2-c]pyridin,

4-(p-Fluorphenyl)-6-methyl-4,5,6,7-tetrahydro-furano-[3,2-c]pyridin,

4-(p-Chlorphenyl)-6-methyl-4,5,6,7-tetrahydro-furano-[3,2-c]pyridin,

2,6-Dimethyl-4-(p-fluorphenyl)-4,5,6,7-tetrahydro-furano[3,2-c]pyridin,

4-(p-Methoxyphenyl)-6-methyl-4,5,6,7-tetrahydro-furano[3,2-c]pyridin,

2,6-Dimethyl-4-(p-tolyl)-4,5,6,7-tetrahydro-furano-[3,2-c]pyridin,

2,6-Dimethyl-4-(p-chlorphenyl)-4,5,6,7-tetrahydro-furano[3,2-c]pyridin,

2,6-Dimethyl-4-(p-bromphenyl)-4,5,6,7-tetrahydro-furano[3,2-c]pyridin,

2,6-Dimethyl-4-(p-methoxyphenyl)-4,5,6,7-tetrahydro-furano[3,2-c]pyridin,

2-Ethyl-4-(p-fluorphenyl)-6-methyl-4,5,6,7-tetrahydro-furano[3,2-c]pyridin,

2-Methyl-4-phenyl-4,5,6,7-tetrahydro-furano[3,2-c]-pyridin,

4-(3,4-Dihydroxyphenyl)-6-methyl-4,5,6,7-tetrahydro-furano[3,2-c]pyridin,

4-(3-Hydroxyphenyl)-6-methyl-4,5,6,7-tetrahydro-furano[3,2-c]pyridin,

4-(3,4-Dihydroxyphenyl)-4,5,6,7-tetrahydro-furano[3,2-c]pyridin,

2-Brom-4-(p-fluorphenyl)-6-methyl-4,5,6,7-tetrahydro-furano[3,2-c]pyridin.

Die Ausgangsverbindungen der allgemeinen Formeln III und VII können nach an sich bekannten Verfahren erhalten werden:
Verbindungen der allgemeinen Formel III können hergestellt werden durch Kondensation eines Furfurylglycinesters mit Chlorameisensäureethylester, Reduktion des entstandenen 4,5,6,7-Tetrahydro-4-oxo-6-ethoxy-carbonyl-furano[2,3-c]pyridins mit Lithiumalanat oder Natriumborhydrid und Abspaltung der Ethoxycarbonylgruppe mit Alkali. Ausgangsstoffe der allgemeinen Formel VII erhält man z.B. durch Umsetzung von Furfurylmethylamin mit p-Methylphenacylbromid und anschließend mit Natriumborhydrid.

Die Endprodukte der allgemeinen Formel I können gewünschtenfalls nach üblichen Methoden in ihre physiologisch unbedenklichen Säureadditionssalze überführt werden. Als Säuren eignen sich hierfür sowohl anorganische Säuren wie Halogenwasserstoffsäuren, Schwefelsäure, Phosphorsäure und Aminosulfonsäure, als auch organische Säuren wie Ameisensäure, Essigsäure, Propionsäure, Milchsäure, Glykolsäure, Glukonsäure, Maleinsäure, Bernsteinsäure, Weinsäure, Benzoesäure, Salicylsäure, Zitronensäure, Ascorbinsäure, Oxalsäure, p-Toluolsulfonsäure oder Oxyethansulfonsäure.

Die neuen Stoffe der allgemeinen Formeln I und II und ihre Säureadditionssalze stellen wertvolle Pharmazeutika dar. In einigen spezifischen Tests hat sich herausgestellt, daß sie starke antidepressive Eigenschaften besitzen und insbesondere eine thymoleptische und zentralanregende Wirkung ausüben.

Ein Test zur Bestimmung der antidepressiven Wirkung ist der Tetrabenazin-Antagonismus, die Aufhebung der durch Tetrabenazin verursachten Ptosis. Der Versuch wird an Mäusen durchgeführt, pro Dosis werden 5 Tiere verwendet. Eine Stunde nach Gabe der Testsubstanz wird den Tieren 40 mg/kg Tetrabenazin verabreicht. Die Beurteilung der Ptosis erfolgt 75 - 120 Minuten nach der Tetrabenazingabe, entsprechend den Angaben von B. Rubin, M. H. Malone et al, J. Pharmacol. Exp. Ther. 120, 125 - 136 (1957).

Bei einem weiteren - biochemischen - Test geht man von der Tatsache aus, daß es bei verschiedenen Depressionsformen in bestimmten Gehirnarealen zu einer Verarmung an biogenen Aminen, vor allem an Noradrenalin, kommt; die biogenen Amine können dadurch vermehrt werden, daß der Uptake in die Neuronen verhindert wird. Eine geeignete Versuchsanordnung zeigt, daß die neuen Verbindungen vor allem die Wiederaufnahme von Noradrenalin in die Neuronen hemmen.
Der Versuch wird, entsprechend den Angaben von A. S. Horn, Mol. Pharmacol. 1971, S. 66 - 80, am homogenisierten isolierten Rattengroßhirn durchgeführt. Die so erhaltenen Synaptosomen werden mit tritiiertem Noradrenalin und verschiedenen Konzentrationen einer Lösung der Testsubstanz in Wasser 10 Minuten bei 37°C inkubiert. Nach Beendigung der Inkubation wird das Medium durch Filtrieren abgetrennt und die Radioaktivität der Synaptosomen gemessen.

Ein Kontrollversuch ohne Testsubstanz läuft zur Bestimmung der Aufnahmemenge der radioaktiven Amine mit.
Als $IC_{50}$ wird diejenige Menge an Testsubstanz in Mol bezeichnet, die ausreicht, um 50 % der Aufnahme (Uptake) zu verhindern.

In neuerer Zeit tritt immer mehr eine Bestimmung des $\alpha_2$-Rezeptor-Antagonismus in den Vordergrund. Der präsynaptische $\alpha_2$-Rezeptor, auch Autorezeptor genannt, reguliert die Noradrenalinfreisetzung, die im Falle einer Blockade des Rezeptors ungebremst erfolgt. Voraussetzung für die Blockade des Rezeptors ist eine hohe Affinität der Testsubstanz zum $\alpha_2$-Rezeptor (s. Greenberg et al, Life Science, Bd. 19, S. 69 (1976)).

Bei Durchführung der oben geschilderten Tests hat sich herausgestellt, daß einige der erfindungsgemäßen Verbindungen eine hohe Wirkung im Tetrabenazintest aufweisen, während andere sich beispielsweise bei der Noradrenalinhemmung beziehungsweise im $\alpha_2$-Rezeptor-Bindungstest als bekannten Antidepressiva überlegen erwiesen haben.

Die erfindungsgemäß erhältlichen Verbindungen können allein oder in Kombination mit anderen erfindungsgemäßen Wirkstoffen, gegebenenfalls auch in Kombination mit weiteren pharmakologisch aktiven Wirkstoffen zur Anwendung gelangen. Geeignete Anwendungsformen sind beispielsweise Tabletten, Kapseln, Zäpfchen, Lösungen, Säfte, Emulsionen oder dispersible Pulver. Entsprechende Tabletten können beispielsweise durch Mischen des oder der Wirkstoffe mit bekannten Hilfsstoffen, beispielsweise inerten Verdünnungsmitteln wie Calciumcarbonat, Calciumphosphat oder Milchzucker, Sprengmitteln wie Maisstärke oder Alginsäure, Bindemitteln wie Stärke oder Gelatine, Schmiermitteln wie Magnesiumstearat oder Talk, und/oder Mitteln zur Erzielung des Depoteffektes wie Carboxymethylen, Carboxymethylcellulose, Celluloseacetatphthalat oder Polyvinylacetat erhalten werden. Die Tabletten können auch aus mehreren Schichten bestehen.

Entsprechend können Dragees durch Überziehen von analog den Tabletten hergestellten Kernen mit üblicherweise in Drageehüllen verwendeten Mitteln, beispielsweise Kollidon oder Schellack, Gummi arabicum, Talk, Titandioxid oder Zucker, hergestellt werden. Zur Erzielung eines Depoteffektes oder zur Vermeidung von Inkompatibilitäten kann der Kern auch aus mehreren Schichten bestehen. Desgleichen kann auch die Drageehülle zur Erzielung eines Depoteffektes aus mehreren Schichten bestehen, wobei die oben bei den Tabletten erwähnten Hilfsstoffe verwendet werden können.

Säfte der erfindungsgemäßen Wirkstoffe beziehungsweise Wirkstoffkombinationen können zusätzlich noch ein Süßungsmittel wie Saccharin, Cyclamat, Glycerin oder Zucker sowie ein geschmacksverbesserndes Mittel, z.B. Aromastoffe wie Vanillin oder Orangenextrakt, enthalten. Sie können außerdem Suspendierhilfsstoffe oder Dickungsmittel wie Natriumcarboxymethylcellulose, Netzmittel, beispielsweise Kondensationsprodukte von Fettalkoholen mit Ethylenoxid, oder Schutzstoffe wie p-Hydroxybenzoate enthalten.

Injektionslösungen werden in üblicher Weise, z.B. unter Zusatz von Konservierungsmitteln wie p-Hydroxybenzoaten oder Stabilisatoren wie Alkalisalzen der Ethylendiamintetraessigsäure, hergestellt und in Injektionsflaschen oder Ampullen abgefüllt.

Die eine oder mehrere Wirkstoffe beziehungsweise Wirkstoffkombinationen enthaltenden Kapseln können beispielsweise hergestellt werden, indem man die Wirkstoffe mit inerten Trägern wie Milchzucker oder Sorbit mischt und in Gelatinekapseln einkapselt.

Geeignete Zäpfchen lassen sich beispielsweise durch Vermischen mit dafür vorgesehenen Trägermitteln wie Neutralfetten oder Polyethylenglykol beziehungsweise dessen Derivaten herstellen.

Die folgenden Beispiele dienen zur näheren Erläuterung der Erfindung:

Beispiel 1

4-(p-Methoxyphenyl)-6-methyl-4,5,6,7-tetrahydro-furano[2,3-c]pyridin

Zu 30 g Anisol in 90 g Methansulfonsäure werden 14,5 g (0,095 Mol) 4-Hydroxy-6-methyl-4,5,6,7-tetrahydro-furano[2,3-c]pyridin gegeben und eine Stunde bei 5 - 10°C gerührt. Die Reaktionsmischung wird auf Eis gegeben und mit wäßriger Ammoniaklösung neutralisiert. Es wird mit Ether extrahiert, die Etherlösung getrocknet und das Lösungsmittel abdestilliert. Der Rückstand wird über Kieselgel (Cyclohexan/Essigester, 1:1) gereinigt und getrennt. Nach Entfernung des Lösungsmittels wird der Rückstand der 1. Fraktion mit etherischer Salzsäure versetzt. Es kristallisiert das Hydrochlorid der Titelverbindung.
Ausbeute:11,0 g (40,8 % d.Th.); Fp. 199 - 200°C.

Die 2. Fraktion enthält das 4-(o-Methoxyphenyl)-6-methyl-4,5,6,7-tetrahydro-furano[2,3-c]pyridin, das nach dem Abdestillieren des Lösungsmittels kristallisiert.
Ausbeute: 1,6 g; Fp. 116 - 117°C.

Analog wurden folgende Endprodukte hergestellt:
ausgehend von 4-Hydroxy-6-methyl-4,5,6,7-tetrahydro-furano[2,3-c]pyridin:

4-(p-Hydroxyphenyl)-6-methyl-4,5,6,7-tetrahydro-furano[2,3-c]pyridin; Fp. 210 - 211°C;

4-(3,4-Dihydroxyphenyl)-4,5,6,7-tetrahydro-furano-[2,3-c]pyridin; Hydrochlorid Fp. 262 - 263°C;

ausgehend von 2,6-Dimethyl-4-hydroxy-4,5,6,7-tetrahydro-furano[2,3-c]pyridin:

2,6-Dimethyl-4-(3,4-dihydroxyphenyl)-4,5,6,7-tetrahydro-furano[2,3-c]pyridin; Hydrochlorid Fp. 237 - 238°C;

2,6-Dimethyl-4-(p-hydroxyphenyl)-4,5,6,7-tetrahydro-furano[2,3-c]pyridin; Hydrochlorid Fp. 222 - 223°C;

2,6-Dimethyl-4-(o-hydroxyphenyl)-4,5,6,7-tetrahydro-furano[2,3-c]pyridin; Hydrochlorid Fp. 259 - 260°C.

Die Ausgangsverbindung 4-Hydroxy-6-methyl-4,5,6,7-tetrahydro-furano[2,3-c]pyridin wurde auf folgendem Weg erhalten:

a) <u>4,5,6,7-Tetrahydro-4-oxo-6-ethoxycarbonyl-furano-[2,3-c]pyridin</u>

Zu 274,5 g (1,5 Mol) N-Furfurylglycinester und 167 g Triethylamin in 2000 ml Methylenchlorid werden unter Eiskühlung 179 g (1,65 Mol) Chlorameisensäureethylester zugetropft. Die Reaktionsmischung wird 1 Stunde bei Raumtemperatur nachgerührt und anschließend mit 500 ml verdünnter Salzsäure ausgeschüttelt. Die organische Phase wird abgetrennt, getrocknet und eingeengt. Die erhaltene Verbindung wird mit 1,5 Mol Kaliumhydroxid in 2000 ml Methanol 1 Stunde auf 50°C erhitzt. Die Reaktionsmischung wird bis zur Kristallisation eingeengt. Die Kristalle werden abgesaugt und in wenig Wasser gelöst. Nach Zugabe von halbkonzentrierter Salzsäure scheidet sich ein Öl ab, das mit Methylenchlorid extrahiert wird. Nach dem Trocknen und Entfernen des Lösungsmittels wird mit 69 ml Thionylchlorid versetzt und 6 Stunden unter Rückfluß zum Sieden erhitzt. Im Vakuum werden alle flüchtigen Bestandteile entfernt.

Der Rückstand wird in 2000 ml Ethylenchlorid gelöst und mit 3 Mol Aluminiumchlorid versetzt. Nach 1 Minute wird die Reaktionsmischung auf Eis gegeben. Mit Methylenchlorid werden die organischen Bestandteile extrahiert. Anschließend wird getrocknet und eingeengt. Der Rückstand wird mit verdünntem Ammoniak versetzt, die Lösung mit Ether extrahiert, getrocknet und der Ether abdestilliert. Die Titelverbindung kristallisiert aus Diisopropylether/Cyclohexan-Gemisch mit einem Fp. von 63 - 64°C.

b) <u>4,5,6,7-Tetrahydro-4-hydroxy-6-methyl-furano-[2,3-c]pyridin</u>

Zu 13 g Lithiumalanat in 300 ml absolutem Tetrahydrofuran werden 52,3 g (0,25 Mol) 4,5,6,7-Tetrahydro-4-oxo-6-ethoxycarbonyl-furano[2,3-c]pyridin gelöst und in 300 ml absolutem Tetrahydrofuran unter Eiskühlung bei 10 - 15°C zugetropft. Nach beendeter Zugabe wird 30 Minuten unter Rückfluß zum Sieden erhitzt. Nach dem Abkühlen wird der Überschuß an Lithiumalanat mit einer wäßrigen Tetrahydrofuran-Lösung (THF: $H_2O$, 90 : 10) zersetzt. Zur Reaktionsmischung werden 1300 ml 40%ige Diammoniumtartratlösung zugegeben. Die organische Phase wird abgetrennt und getrocknet. Nach Entfernen des Lösungsmittels im Vakuum wird der Rückstand über Kieselgel filtriert. Man erhält das 4,5,6,7-Tetrahydro-4-hydroxy-6-methyl-furano-[2,3-c]pyridin Hydrochlorid vom Fp. 169 - 170°C.

Auf diesem Weg wurden ferner das 2,6-Dimethyl-4-hydroxy-furano[2,3-c]pyridin, Fp. des Hydrochlorids 189 - 190°C, und das 2-Ethyl-4-hydroxy-6-methyl-furano[2,3-c]pyridin, Fp. des Hydrochlorids 176-178°C, erhalten.

Ausgehend von 4-Hydroxy-4,5,6,7-tetrahydro-furano-[2,3-c]pyridin:

4-(o-Hydroxyphenyl)-4,5,6,7-tetrahydro-furano[2,3-c]-pyridin; Fp. 186 - 187°C;

ausgehend von 2-Methyl-4-hydroxy-4,5,6,7-tetrahydro-furano[2,3-c]pyridin:

2-Methyl-4-(p-methoxyphenyl)-4,5,6,7-tetrahydro-furano[2,3-c]pyridin; Hydrochlorid Fp. 255 - 256°C.

Das als Ausgangsverbindung verwendete 4-Hydroxy-4,5,6,7-tetrahydro-furano[2,3-c]pyridin wurde wie folgt hergestellt:

36 g (0,17 Mol) 4,5,6,7-Tetrahydro-4-oxo-6-ethoxycarbonyl-furano[2,3-c]pyridin werden in 100 ml Ethanol gelöst und mit 10 g (0,25 Mol) Natriumborhydrid 30 Minuten bei 40 - 50°C gerührt. Das Lösungsmittel wird im Vakuum entfernt und der Rückstand mit 200 ml Wasser versetzt und mit Ether extrahiert. Nach dem Trocknen wird der Ether abdestilliert.

Der Rückstand, 4,5,6,7-Tetrahydro-4-hydroxy-6-ethoxycarbonyl-furano[2,3-c]pyridin, wird in 500 ml Methanol gelöst, mit 44 g Kaliumhydroxid versetzt und 48 Stunden im Autoklaven auf 120°C erhitzt. Nach dem Abkühlen wird das Lösungsmittel im Vakuum abdestilliert, der Rückstand in Methylenchlorid gelöst und im Magnesiumsulfat getrocknet. Anschließend werden die festen Bestandteile abgesaugt und das Lösungsmittel abdestilliert. Der Rückstand wird in Ethanol gelöst. Nach Zugabe von etherischer Salzsäure kristallisiert das Hydrochlorid der Titelverbindung aus.
Ausbeute: 22 g (73,5 % d.Th.); Fp. 167 - 168°C.

Entsprechend erhält man das 2-Methyl-4-hydroxy-4,5,6,7-tetrahydro-furano[2,3-c]pyridin-Hydrochlorid vom Fp. 200 - 201°C (Zers.).

Beispiel 2

4-(p-Bromphenyl)-2,6-dimethyl-4,5,6,7-tetrahydro-furano[2,3-c]pyridin

6 g (0,03 Mol) 2,6-Dimethyl-4-hydroxy-4,5,6,7-tetrahydro-furano[2,3-c]pyridin und 10 ml Brombenzol werden in 60 ml Methylenchlorid gelöst, mit 9 g Aluminiumchlorid versetzt und 15 Minuten gerührt. Das Reaktionsgemisch wird auf Eis gegeben, mit Natronlauge alkalisch gestellt und mit Ethylenchlorid extrahiert. Das Lösungsmittel wird abdestilliert und der Rückstand über Kieselgel (Cyclohexan/Essigester, 3 : 1) gereinigt und getrennt. Aus dem Rückstand der 1. Fraktion kristallisiert nach Zugabe von etherischer Salzsäurelösung das Hydrochlorid der Titelverbindung.
Ausbeute: 2,5 g (24,7 % d.Th.); Fp. 203 - 204°C.

Aus dem Rückstand der 2. Fraktion kristallisiert nach Zugabe von etherischer Salzsäurelösung das Hydrochlorid des 4-(o-Bromphenyl)-2,6-dimethyl-4,5,6,7-tetrahydro-furano[2,3-c]pyridins.
Ausbeute: 5,0 g (49,4 % d.Th.); Fp. 252 - 253°C.

Analog wurden folgende Endprodukte erhalten:

ausgehend von 4-Hydroxy-6-methyl-4,5,6,7-tetrahydro-furano[2,3-c]pyridin:

4-(p-Tolyl)-6-methyl-4,5,6,7-tetrahydro-furano[2,3-c]-pyridin; Maleinat Fp. 180 - 181°C;

6-Methyl-4-phenyl-4,5,6,7-tetrahydro-furano[2,3-c]-pyridin; Maleinat Fp. 174 - 175°C;

ausgehend von 2,6-Dimethyl-4-hydroxy-4,5,6,7-tetrahydro-furano[2,3-c]pyridin:

2,6-Dimethyl-4-(o-bromphenyl)-4,5,6,7-tetrahydro-furano[2,3-c]pyridin; Hydrochlorid Fp. 203 - 204°C;

2,6-Dimethyl-4-(p-chlorphenyl)-4,5,6,7-tetrahydro-furano[2,3-c]pyridin; Hydrochlorid Fp. 241 - 242°C;

2,6-Dimethyl-4-(o-chlorphenyl)-4,5,6,7-tetrahydro-furano[2,3-c]pyridin; Hydrochlorid Fp. 223 - 224°C;

2,6-Dimethyl-4-(p-fluorphenyl)-4,5,6,7-tetrahydro-furano[2,3-c]pyridin; Hydrochlorid Fp. 209 - 210°C;

2,6-Dimethyl-4-(o-fluorphenyl)-4,5,6,7-tetrahydro-furano[2,3-c]pyridin; Hydrochlorid Fp. 242 - 243°C;

ausgehend von 4-Hydroxy-4,5,6,7-tetrahydro-furano-[2,3-c]pyridin:

4-(p-Fluorphenyl)-4,5,6,7-tetrahydro-furano[2,3-c]-pyridin; Maleinat Fp. 128 - 129°C;

ausgehend von 2-Methyl-4-hydroxy-4,5,6,7-tetrahydro-furano[2,3-c]pyridin:

2-Methyl-4-(p-bromphenyl)-4,5,6,7-tetrahydro-furano-[2,3-c]pyridin; Hydrochlorid Fp. 273 - 274°C;

2-Methyl-4-(o-bromphenyl)-4,5,6,7-tetrahydro-furano-[2,3-c]pyridin; Hydrochlorid Fp. 229 - 230°C;

2-Pyrrolidinomethyl-4-(p-methoxyphenyl)-6-methyl-4,5,6,7-tetrahydro-furano[2,3-c]pyridin; Hydrochlorid Fp. 184 - 185°C (Zers.);

ausgehend von 2-Methyl-4-hydroxy-6-ethyl-4,5,6,7-tetrahydro-furano[2,3-c]pyridin:

2-Methyl-4-phenyl-6-ethyl-4,5,6,7-tetrahydro-furano-[2,3-c]pyridin; Hydrochlorid Fp. 185 - 186°C (Zers.);

ausgehend von 2 Methyl-4-hydroxy-6-n-propyl-4,5,6,7-tetrahydro-furano[2,3-c]pyridin:

2-Methyl-4-phenyl-6-n-propyl-4,5,6,7-tetrahydro-furano[2,3-c]pyridin; Oxalat Fp. 166 - 168°C;

ausgehend von 2-Methyl-4-hydroxy-6-n-butyl-4,5,6,7-tetrahydro-furano[2,3-c]pyridin:

2-Methyl-4-phenyl-6-n-butyl-4,5,6,7-tetrahydro-furano[2,3-c]pyridin; Oxalat Fp. 114 - 115°C.

<u>2-Methyl-4-hydroxy-6-butyl-tetrahydro-furano-[2,3-c]pyridin</u>

Die Ausgangsverbindung erhält man wie folgt:

3 g (0,02 Mol) 4,5,6,7-Tetrahydro-4-hydroxy-6-ethoxy-carbonyl-furano[2,3-c]pyridin werden in 50 ml Ethanol gelöst, mit 3 ml Triethylamin und 3 g n-Butylbromid versetzt und 2 Stunden unter Rückfluß zum Sieden erhitzt. Die Reaktionsmischung wird im Vakuum weitgehend eingeengt und mit Wasser versetzt. Die wäßrige Lösung wird mit Ether extrahiert. Der Ether wird abdestilliert und zurück bleibt die Titelverbindung als hellgelbes Öl.
Ausbeute: 2,6 g (63,4 % d.Th.); Fp. des Hydrochlorids 165 - 166°C.

Analog wird das 1-Methyl-4-hydroxy-6-ethyl-4,5,6,7-tetrahydro-furano[2,3-c]pyridin, Fp. des Hydrochlorids 179 - 180°C, und das 1-Methyl-4-hydroxy-6-n-propyl-4,5,6,7-tetrahydro-furano[2,3-c]pyridin, Fp. des Hydrochlorids 170 - 171°C, erhalten.

Beispiel 3

4-(3,4-Dihydroxyphenyl)-4,5,6,7-tetrahydro-furano-[2,3-c]pyridin

Zu einer Lösung von 13 g 4-Hydroxy-4,5,6,7-tetrahydro-furano[2,3-c]pyridin und 22 g Triethylamin in 200 ml Ethanol werden 16 g o-Chlorbenzylchlorid gegeben und eine Stunde unter Rückfluß zum Sieden erhitzt. Die flüchtigen Anteile werden im Vakuum weitgehend entfernt und der Rückstand mit Wasser versetzt. Die Lösung wird mit Ether extrahiert und getrocknet. Nach Abdestillieren des Lösungsmittels verbleiben 23 g eines hellgelben Öls, das in 200 ml Methylenchlorid gelöst wird und zu einer Lösung aus 14 g Brenzcatechin und 100 ml Methansulfonsäure in 200 ml Methylenchlorid getropft wird. Nach 30 Minuten wird auf Eis gegeben und mit konzentriertem Ammoniak neutralisiert. Die organische Phase wird abgetrennt und getrocknet. Nach Abdestillieren des Lösungsmittels kristallisiert das 4-Hydroxy-6-(o-chlorbenzyl)-4,5,6,7-tetrahydro-furano[2,3-c]pyridin (16 g).

Die Entbenzylierung erfolgt in Methanol bei Raumtemperatur unter 5bar mit Palladium/Kohle. Nach dem Absaugen des Katalysators und Abdestillieren des Lösungsmittels kristallisiert der Rückstand nach Zugabe von etherischer Salzsäure.
Ausbeute: 9 g (77 % d.Th.); Fp. 243 - 244°C (Zers.) (aus Isopropanol).

Analog wurden die folgenden Hydrochloride der Endprodukte hergestellt:

2-Methyl-4-(3,4-dihydroxyphenyl)-4,5,6,7-tetrahydro-furano[2,3-c]pyridin; Fp. 214 - 215°C (Zers.);

2-Methyl-4-(1,4-benzodioxanyl)-4,5,6,7-tetrahydro-furano[2,3-c]pyridin; Fp. 241 - 242°C (Zers.).

Beispiel 4

4-(p-Methoxyphenyl)-6-methyl-2-pyrrolidinomethyl-4,5,6,7-tetrahydro-furano[2,3-c]pyridin

2 g (0,00825 Mol) 4-(p-Methoxyphenyl)-6-methyl-4,5,6,7-tetrahydro-furano[2,3-c]pyridin und 1,5 g Methylenpyrrolidiniumchlorid werden in 30 ml absolutem Acetonitril gelöst und 30 Minuten zum Sieden erhitzt. Beim Abdestillieren des Lösungsmittels im Vakuum fällt das Hydrochlorid der Titelverbindung aus.
Ausbeute: 1,6 g (52 % d.Th.); Fp. 184 - 185°C (Zers.) (aus Isopropanol).

Beispiel 5

2-Methyl-4-p-acetyloxyphenyl-6-methyl-4,5,6,7-tetrahydro-furano[2,3-c]pyridin

2,5 g (0,008 Mol) 2,6-Dimethyl-4-(p-hydroxyphenyl)-4,5,6,7-tetrahydro-furano[2,3-c]pyridin werden in 25 ml Acetanhydrid 30 Minuten auf 100°C erhitzt. Anschließend wird mit Ammoniak neutralisiert und mit Essigester extrahiert. Die organische Phase wird getrocknet und im Vakuum das Lösungsmittel abdestilliert. Durch Zugabe von etherischer Salzsäure zum Rückstand kristallisiert das Hydrochlorid der Titelverbindung.
Ausbeute: 2,8 g (88 % d.Th.); Fp. 210 - 211°C.

Beispiel 6

2,6-Dimethyl-4-p-tolyl-4-hydroxy-4,5,6,7-tetrahydro-furano[2,3-c]pyridin

In einer Lösung von 0,03 Mol p-Tolylmagnesiumbromid in 25 ml absolutem Tetrahydrofuran wird bei -10°C eine Lösung von 1,5 g (0,009 Mol) 2,6-Dimethyl-4-oxo-4,5,6,7-tetrahydro-furano[2,3-c]pyridin in 10 ml absolutem Tetrahydrofuran getropft. Nach der Reaktion wird der Ansatz auf Eis gegeben und mit Ether extrahiert. Nach dem Trocknen wird der Ether abdestilliert und der Rückstand in Ethanol gelöst. Nach Zugabe von etherischer Salzsäure kristallisiert das Hydrochlorid der Titelverbindung.
Ausbeute: 2,3 g (86 % d.Th.); Fp. 180 - 181°C (aus Ethanol).

Analog wurden die folgenden Endprodukte in Form ihres Hydrochlorides erhalten:

ausgehend von 2,6-Dimethyl-4-oxo-4,5,6,7-tetrahydro-furano[2,3-c]pyridin:

2,6-Dimethyl-4-hydroxy-4-(p-bromphenyl)-4,5,6,7-tetrahydro-furano[2,3-c]pyridin; Fp. 196 - 197°C (Zers.);

2,6-Dimethyl-4-hydroxy-4-(m-methoxyphenyl)-4,5,6,7-tetrahydro-furano[2,3-c]pyridin; Fp. 173 - 174°C (Zers.);

2,6-Dimethyl-4-hydroxy-4-(p-ethylphenyl)-4,5,6,7-tetrahydro-furano[2,3-c]pyridin; Fp. 181 - 182°C;

2,6-Dimethyl-4-hydroxy-4-(p-chlorphenyl)-4,5,6,7-tetrahydro-furano[2,3-c]pyridin; Fp. 199 - 200°C;

Die Ausgangsverbindung, 2,6-Dimethyl-4-oxo-4,5,6,7-tetrahydro-furano[2,3-c]pyridin, wurde wie folgt erhalten:

Zu einer Lösung von 10 g (0,05 Mol) 2,6-Dimethyl-4-hydroxy-4,5,6,7-tetrahydro-furano[2,3-c]pyridin und 100 ml Cyclohexanon in 1 l absolutem Toluol werden 22,8 g Aluminiumisopropylat in 500 ml Toluol zugetropft. Die Reaktionsmischung wird 2 Stunden unter Rückfluß zum Sieden erhitzt. Nach dem Abkühlen wird mit 200 ml 40%iger Diammoniumtartratlösung versetzt. Die organische Phase wird abgetrennt und getrocknet. Nach dem Abdestillieren des Lösungsmittels bleibt die Titelverbindung als gelbes Öl zurück.
Ausbeute: 5,5 g (66,6 % d.Th.); Fp. des Hydrochlorids 222 - 223°C.

Analog wird aus 4-Hydroxy-6-methyl-4,5,6,7-tetrahydro-furano[2,3-c]pyridin das 4-Oxo-4,5,6,7-tetrahydro-furano[2,3-c]pyridin, Fp. des Hydrochlorids 243 - 244°C, erhalten.

Ausgehend von 4-Oxo-6-methyl-4,5,6,7-tetrahydro-furano[2,3-c]pyridin:

4-Hydroxy-4-(p-bromphenyl)-6-methyl-4,5,6,7-tetrahydro-furano[2,3-c]pyridin; Hydrochlorid Fp. 212-213°C (Zers.).

Beispiel 7

4-Methoxy-4-p-bromphenyl-6-methyl-4,5,6,7-tetrahydro-furano[2,3-c]pyridin

2 g (0,005 Mol) 4-Hydroxy-4-(p-bromphenyl)-6-methyl-4,5,6,7-tetrahydro-furano[2,3-c]pyridin werden in 40 ml Bortrifluorid-Methanol-Gemisch (20 % $BF_3$) 4 Stunden

auf 50°C erwärmt. Anschließend wird auf Eis gegeben und mit Ammoniak neutralisiert. Das Gemisch wird mit Ether extrahiert und der Ether im Vakuum abdestilliert. Der Rückstand wird über Kieselgel/Cyclohexan: Essigester (75 : 25) gereinigt.
Ausbeute: 1 g (56 % d.Th.); Fp. 151 - 152°C (Zers.).

Beispiel 8

2-Hydroxymethyl-4-p-tolyl-6-methyl-4,5,6,7-tetrahydro-furano[2,3-c]pyridin

Zu 200 mg Lithiumalanat in 10 ml absolutem Tetrahydrofuran werden bei 15°C 1,2 g (0,0041 Mol) 2-Carbethoxy-4-p-tolyl-6-methyl-4,5,6,7-tetrahydro-furano[2,3-c]-pyridin, gelöst in 10 ml Tetrahydrofuran, zugetropft. Es wird 30 Minuten bei Raumtemperatur gerührt. Der Überschuß an Lithiumalanat wird mit wäßrigem Tetrahydrofuran zersetzt. Anschließend werden zum Reaktionsgemisch 20 ml 40%ige Diammoniumtartratlösung zugegeben. Die organische Phase wird abgetrennt und getrocknet. Das Lösungsmittel wird im Vakuum abdestilliert. Der Rückstand wird über Kieselgel (Cyclohexan : Essigester 1 : 3) filtriert. Nach Zugabe von alkoholischer Maleinsäure kristallisiert das Maleinat der Titelverbindung.
Ausbeute: 1,1 g (71 % d.Th.); Fp. 94 - 95°C.

Analog wurde, ausgehend von 2-Carbethoxy-4-p-tolyl-4-hydroxy-4,5,6,7-tetrahydro-furano[2,3-c]pyridin, durch Lithiumalanatreduktion das 2-Hydroxymethyl-4-p-tolyl-4-hydroxy-4,5,6,7-tetrahydro-furano[2,3-c]pyridin-maleinat, Fp. 156 - 157°C, hergestellt.

Die Ausgangsverbindung wurde auf folgendem Weg erhalten:

a) 2-Carbethoxy-4-oxo-6-benzyl-4,5,6,7-tetrahydro-furano[2,3-c]pyridin

110 g (0,5 Mol) N-Benzylpiperidindion und 32,5 g Kaliumhydroxyd werden in 800 ml Ethanol gelöst. Zu dieser Lösung werden 70 g (0,51 Mol) Chlorformylessigester gegeben und 10 Stunden bei Raumtemperatur gerührt. Das Lösungsmittel wird im Vakuum weitgehend abdestilliert und der Rückstand mit Wasser versetzt. Die Reaktionsmischung wird mit 1 l Methylenchlorid extrahiert und getrocknet. Zu dieser Lösung wird unter Eiskühlung 150 ml Methansulfonsäure und 50 ml Methansulfonsäurechlorid getropft und 4 Stunden bei Raumtemperatur gerührt. Anschließend wird die Reaktionsmischung auf Eis gegeben und mit konzentriertem Ammoniak neutralisiert. Die organische Phase wird abgetrennt und getrocknet. Nach dem Abdestillieren des Lösungsmittels wird der Rückstand in wenig Aceton gelöst. Nach Zugabe von etherischer Salzsäure kristallisiert das Hydrochlorid der Titelverbindung.
Ausbeute: 55 g (40 % d.Th.); Fp. 175 - 176°C (aus Ethanol).

b) 2-Carbethoxy-4-p-tolyl-4-hydroxy-4,5,6,7-tetrahydro-furano[2,3-c]pyridin

0,1 Mol 4-Methyl-phenylmagnesiumbromid in 60 ml absolutem Tetrahydrofuran werden unter Eiskühlung zu einer Lösung von 15 g (0,045 Mol) 2-Carbethoxy-4-oxo-6-benzyl-4,5,6,7-tetrahydro-furano[2,3-c]pyridin in 60 ml Tetrahydrofuran getropft und 30 Minuten

bei $10^{o}C$ gerührt. Das Reaktionsgemisch wird auf Eis gegeben. Nach Zugabe von 50 ml 2 n Salzsäure kristallisiert das Hydrochlorid des N-Benzyl-Derivates der Titelverbindung (14,5 g, Fp.194-196°C). Die Entbenzylierung wird in 140 ml Ethanol bei 30°C unter 5bar mit Palladium/Kohle durchgeführt. Nach dem Absaugen des Katalysators wird im Vakuum das Lösungsmittel abdestilliert. Die Titelverbindung kristallisiert in Ether.
Ausbeute: 7,6 g (75 % d.Th.); Fp. 160 - 161°C.

c) <u>2-Carbethoxy-4-p-tolyl-6-methyl-4,5,6,7-tetrahydro-furano[2,3-c]pyridin</u>

3,7 g (0,0123 Mol) 2-Carbethoxy-4-p-tolyl-4-hydroxy-4,5,6,7-tetrahydro-furano[2,3-c]pyridin werden in 40 ml Ethylenchlorid gelöst und langsam mit 4 ml Thionylchlorid versetzt und 30 Minuten bei Raumtemperatur gerührt. Die flüchtigen Bestandteile werden im Vakuum abdestilliert. Der Rückstand wird mit Eis versetzt und mit Ammoniak neutralisiert. Die wäßrige Phase wird mit Methylenchlorid extrahiert, die organische Phase getrocknet und im Vakuum eingeengt. Nach Zugabe von Isopropylether zum Rückstand kristallisieren 3 g (84,5 %) 2-Carbethoxy-4-p-tolyl-furano[2,3-c]pyridin, Fp. 116 - 117°C. Dieses wird in 28 ml Tetrahydrofuran gelöst und mit 5 g Natriumcarbonat und 10 g Methyljodid 6 Stunden am Rückfluß erhitzt. Nach dem Abkühlen wird Methylenchlorid und Wasser zugegeben bis sich die organische Phase separiert.
Die organische Phase wird abgetrennt, getrocknet und im Vakuum eingeengt. Das Methylpyridinium-jodid (2,8 g) scheidet sich kristallin ab. Die Kristalle werden mit Ether gewaschen. Fp. 214 - 215°C (Zers.).

Das entstandene Pyridinium-jodid wird in 70 ml Ethanol gelöst und bei 70°C unter Platinoxyd-Katalyse hydriert. Nach Absaugen des Katalysators wird mit Ammoniak alkalisch gestellt und mit Methylenchlorid extrahiert. Nach dem Abdestillieren des Lösungsmittels bleibt die Titelverbindung als hellgelbes Öl zurück.
Ausbeute: 1,2 g (66,5 % d.Th. bezogen auf das Methyl-pyridinium-jodid.

Beispiel 9

4-p-Tolyl-6-methyl-4,5,6,7-tetrahydro-furano[3,2-c]-pyridin

20,0 g (0,08 Mol) 2-Furfuryl-2-methyl-amino-1-p-methyl-phenyl-ethanol werden in 100 ml Ethylenchlorid gelöst und mit 100 ml Trifluoressigsäure 3 Stunden zum Sieden erhitzt. Die Reaktionsmischung wird auf Eis gegeben und mit konzentriertem Ammoniak neutralisiert. Die organischen Bestandteile werden mit Ether extrahiert. Nach dem Trocknen wird der Ether im Vakuum abdestilliert. Der Rückstand wird über Kieselgel/Cyclohexan: Essigester (1:1) gereinigt. Nach dem Abdestillieren des Lösungsmittels im Vakuum wird der Rückstand in Aceton gelöst. Nach Zugabe von etherischer Salzsäure kristallisiert das Hydrochlorid der Titelverbindung.
Ausbeute: 4,5 g (19 % d.Th.); Fp. 248 - 249°C.

Analog wurden die folgenden Endprodukte erhalten:

4-Phenyl-4,5,6,7-tetrahydro-furano[3,2-c]pyridin,
Fp. 237 - 238°C, (Hydrochlorid)

2,6-Dimethyl-4-phenyl-4,5,6,7-tetrahydro-furano[3,2-c]-pyridin, Fp. 188 - 189°C, (Hydrochlorid)

4-(p-Tolyl)-6-methyl-4,5,6,7-tetrahydro-furano[3,2-c]-pyridin, Fp. 248 - 249°C, (Hydrochlorid)

4-Phenyl-6-methyl-4,5,6,7-tetrahydro-furano[3,2-c]-pyridin, Fp. 237 - 238°C, (Hydrochlorid)

4-(p-Bromphenyl-6-methyl-4,5,6,7-tetrahydro-furano-[3,2-c]pyridin, Fp. 259 - 260°C, (Hydrochlorid)

4-(p-Fluorphenyl)-6-methyl-4,5,6,7-tetrahydro-furano-[3,2-c]pyridin, Fp. 224 - 225°C, (Hydrochlorid)

4-(p-Chlorphenyl)-6-methyl-4,5,6,7-tetrahydro-furano-[3,2-c]pyridin, Fp. 239 - 240°C, (Hydrochlorid)

2,6-Dimethyl-4-(p-fluorphenyl)-4,5,6,7-tetrahydro-furano[3,2-c]pyridin, Fp. 202 - 203°C, (Hydrochlorid)

4-(p-Methoxyphenyl)-6-methyl-4,5,6,7-tetrahydro-furano[3,2-c]pyridin, Fp. 221 - 222°C, (Hydrochlorid)

2,6-Dimethyl-4-(p-tolyl)-4,5,6,7-tetrahydro-furano-[3,2-c]pyridin, Fp. 224 - 225°C, (Hydrochlorid)

2,6-Dimethyl-4-(p-chlorphenyl)-4,5,6,7-tetrahydro-furano[3,2-c]pyridin, Fp. 177 - 178°C, (Hydrochlorid)

2,6-Dimethyl-4-(p-bromphenyl)-4,5,6,7-tetrahydro-furano[3,2-c]pyridin, Fp. 174 - 175°C, (Hydrochlorid)

2,6-Dimethyl-4-(p-methoxyphenyl)-4,5,6,7-tetrahydro-furano[3,2-c]pyridin, Fp. 240 - 241°C, (Hydrochlorid)

2-Ethyl-4-(p-fluorphenyl)-6-methyl-4,5,6,7-tetrahydro-furano[3,2-c]pyridin, Fp. 214 - 215°C, (Hydrochlorid)

2-Methyl-4-phenyl-4,5,6,7-tetrahydro-furano[3,2-c]-pyridin, Fp. 284 - 286°C (Zers.), (Hydrochlorid)

4-(3,4-Dihydrophenyl)-4,5,6,7-tetrahydro-furano-[3,2-c]pyridin, Fp. 254 - 255°C, (Hydrochlorid)

4-(3-Hydroxyphenyl)-6-methyl-4,5,6,7-tetrahydro-furano[3,2-c]pyridin, Fp. 251 - 252°C, (Hydrochlorid).

2-Furfuryl-2-methyl-amino-1-p-methylphenylethanol

Die Ausgangsverbindung erhält man wie folgt:

Eine Lösung von 10 g (0,082 Mol) Furfurylmethylamin in 50 ml Ethanol und 10 g Triethylamin wird mit 17 g p-Methylphenacylbromid versetzt und 2 Stunden gerührt. Das ausgefallene Triethylamin Hydrochlorid wird abgesaugt. Die Mutterlauge wird mit 4 g Natriumborhydrid versetzt und 1 Stunde bei Raumtemperatur gerührt. Anschließend wird mit Wasser versetzt und mit Ether extrahiert. Die Etherlösung wird getrocknet und der Ether im Vakuum abdestilliert. Das 2-Furfuryl-2-methyl-amino-1-p-methylphenylethanol bleibt als Öl zurück. Ausbeute: 20 g (99 % d.Th.).

## Formulierungsbeispiele

a) Dragees

1 Drageekern enthält:

| | |
|---|---|
| Wirkstoff gemäß der Erfindung | 25,0 mg |
| Milchzucker | 50,0 mg |
| Maisstärke | 22,0 mg |
| Gelatine | 2,0 mg |
| Magnesiumstearat | 1,0 mg |
| | 100,0 mg |

Herstellung:

Die Mischung der Wirksubstanz mit Milchzucker und Maisstärke wird mit einer 10%igen wäßrigen Gelatinelösung durch ein Sieb mit 1 mm Maschenweite granuliert, bei 40°C getrocknet und nochmals durch ein Sieb getrieben. Das so erhaltene Granulat wird mit Magnesiumstearat gemischt und verpreßt. Die so erhaltenen Kerne werden in üblicher Weise mit einer Hülle überzogen, die mit Hilfe einer wäßrigen Suspension von Zucker Titandioxyd, Talkum und Gummi arabicum aufgebracht wird. Die fertigen Dragees werden mit Bienenwachs poliert.
Dragee-Endgewicht: 200 mg.

b) Tabletten

| | |
|---|---|
| Wirkstoff gemäß der Erfindung | 10,0 mg |
| Milchzucker | 40,0 mg |
| Maisstärke | 44,0 mg |
| lösliche Stärke | 5,0 mg |
| Magnesiumstearat | 1,0 mg |
| | 100,0 mg |

Herstellung:

Wirkstoff und Magnesiumstearat werden mit einer wäßrigen Lösung der löslichen Stärke granuliert, das Granulat getrocknet und innig mit Milchzucker und Maisstärke vermischt. Das Gemisch wird sodann zu Tabletten von 100 mg Gewicht verpreßt, die je 10 mg Wirkstoff enthalten.

c) Suppositorien

1 Zäpfchen enthält:

| | |
|---|---|
| Wirkstoff gemäß der Erfindung | 10,0 mg |
| Zäpfchenmasse | 1.690,0 mg |

Herstellung:

Die feingepulverte Substanz wird mit Hilfe eines Eintauch-Homogenisators in die geschmolzene und auf 40°C abgekühlte Zäpfchenmasse eingerührt. Die Masse wird bei 35°C in leicht vorgekühlte Formen gegossen.

d) Ampullen (Injektionslösungen)

Zusammensetzung:

| | |
|---|---|
| Wirkstoff gemäß der Erfindung | 5,0 Gew.-Teile |
| Natriumpyrosulfit | 1,0 Gew.-Teile |
| Dinatriumsalz der Ethylendiamin-tetraessigsäure | 0,5 Gew.-Teile |
| Natriumchlorid | 8,5 Gew.-Teile |
| doppelt destilliertes Wasser ad | 1.000,0 Gew.-Teile |

Herstellung:

Der Wirkstoff und die Hilfsstoffe werden in einer ausreichenden Menge Wasser gelöst und mit der notwendigen Menge Wasser auf die gewünschte Konzentration gebracht. Die Lösung wird filtriert und unter aseptischen Bedingungen in 1 ml Ampullen abgefüllt. Zuletzt werden die Ampullen sterilisiert und verschlossen. Jede Ampulle enthält 5,0 mg Wirkstoff.

## Patentansprüche

1. Neue 4-Phenyl-tetrahydro-furano-pyridine der allgemeinen Formeln

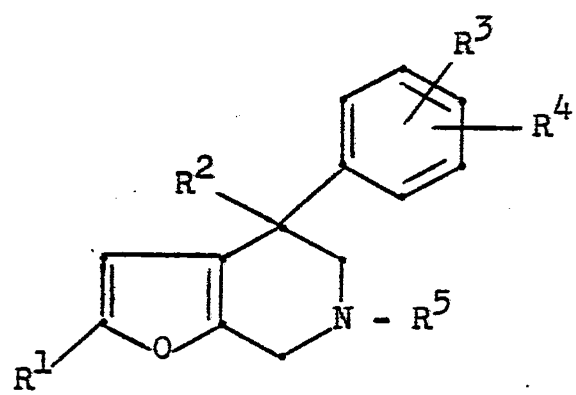

(I)

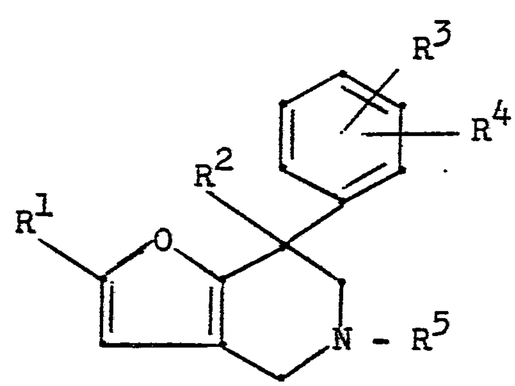

(II)

worin

$R^1$ Wasserstoff, Fluor, Chlor, Brom, eine Alkylgruppe mit 1 - 4 Kohlenstoffatomen, eine Hydroxyalkylgruppe mit 1 - 2 Kohlenstoffatomen, eine Alkoxyalkylgruppe, wobei jeder Alkylrest 1 - 2 Kohlenstoffatome enthalten kann, die Pyrrolidinomethylgruppe oder eine Dialkylaminomethylgruppe, wobei jeder Alkylrest 1 - 2 Kohlenstoffatome enthalten kann;

$R^2$ Wasserstoff, Hydroxy, Methoxy oder Ethoxy;

$R^3$ und $R^4$, die gleich oder verschieden sein können, Fluor, Chlor, Brom, Methyl, Ethyl, Hydroxy, Methoxy, Acetoxy, Benzyloxy oder

$R^3$ und $R^4$ bilden zusammen mit dem Phenylring eine 3,4-Benzodioxanylgruppe;

$R^5$ Wasserstoff, einen Alkylrest mit 1 - 4 Kohlenstoffatomen, den Benzyl- oder o-Chlorbenzylrest bedeuten mit der Maßgabe, daß in Formel II $R^2$ Wasserstoff und $R^1$ nicht Hydroxyalkyl bedeutet sowie deren physiologisch verträgliche Säureadditionssalze.

2. Neue 4-Phenyl-tetrahydro-furano-pyridine der allgemeinen Formel

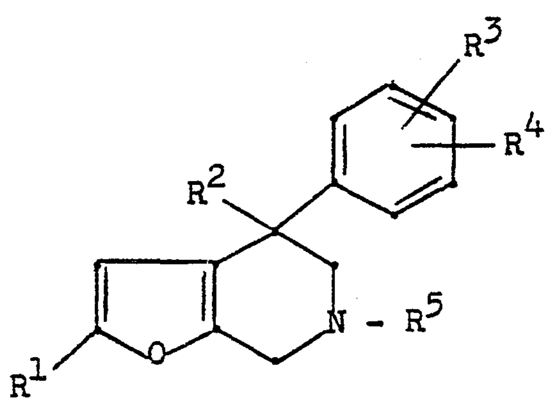

(I)

worin
$R^1$ Wasserstoff oder die Methylgruppe,
$R^2$ Wasserstoff oder die Hydroxygruppe,
$R^3$ und $R^4$, die gleich oder verschieden sein können, Wasserstoff, Hydroxy oder Brom oder zusammen mit dem Phenylring die Benzodioxanylgruppe und
$R^5$ Wasserstoff oder die Methylgruppe bedeuten und deren physiologisch verträgliche Säureadditionssalze.

3. Neue 4-Phenyl-tetrahydro-furano-pyridine der allgemeinen Formel

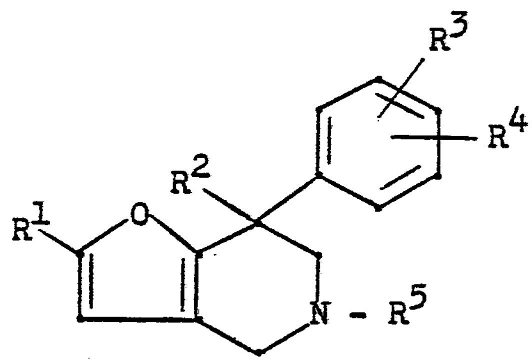
(II)

worin
$R^1$ Wasserstoff oder die Methylgruppe,
$R^2$ Wasserstoff,
$R^3$ und $R^4$, die gleich oder verschieden sein können, Wasserstoff, Hydroxy oder Brom oder zusammen mit dem Phenylring die Benzodioxanylgruppe und
$R^5$ Wasserstoff oder die Methylgruppe bedeuten und deren physiologisch verträgliche Säureadditionssalze.

4. 2,6-Dimethyl-4-hydroxy-4-p-bromphenyl-4,5,6,7-tetrahydro-furano[2,3-c]pyridin und dessen physiologisch verträgliche Säureadditionssalze.

5. 2,6-Dimethyl-4-(3,4-dihydroxyphenyl)-4,5,6,7-tetrahydro-furano[2,3-c]pyridin und dessen physiologisch verträgliche Säureadditionssalze.

6. 4-(3,4-Dihydroxyphenyl)-4,5,6,7-tetrahydro-furano-[2,3-c]pyridin und dessen physiologisch verträgliche Säureadditionssalze.

7. 4-(3,4-Dihydroxyphenyl)-6-methyl-4,5,6,7-tetrahydro-furano[2,3-c]pyridin und dessen physiologisch verträgliche Säureadditionssalze.

8. 2-Methyl-4-benzodioxanyl-4,5,6,7-tetrahydro-furano-[2,3-c]pyridin und dessen physiologisch verträgliche Säureadditionssalze.

9. Verfahren zur Herstellung neuer 4-Phenyl-tetrahydro-furano[2,3-c]pyridine der allgemeinen Formel I gemäß Anspruch 1, worin $R^2$ Wasserstoff bedeutet, dadurch gekennzeichnet, daß man ein 4,5,6,7-Tetrahydro-4-hydroxy-furano[2,3-c]pyridin der allgemeinen Formel

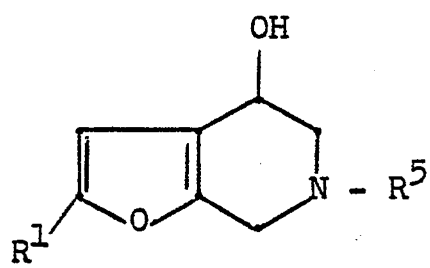

(III)

worin
$R^1$ und $R^5$ die oben angegebene Bedeutung haben, mit einem Benzolderivat der allgemeinen Formel

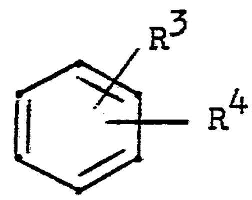

(IV)

worin
$R^3$ und $R^4$ die oben angeführte Bedeutung haben, umsetzt, und daß man ein so erhaltenes Endprodukt gegebenenfalls in üblicher Weise in ein physiologisch verträgliches Säureadditionssalz überführt.

10. Verfahren nach Anspruch 9, dadurch gekennzeichnet, daß man nach beendeter Umsetzung solche erhaltenen Endprodukte der allgemeinen Formel I, in denen nur einer der Reste $R^3$ und $R^4$ eine andere Bedeutung als Wasserstoff hat, durch an sich bekannte Maßnahmen, wie durch Säulenchromatographie, in das entsprechende o- und das p-Isomere auftrennt.

11. Verfahren zur Herstellung neuer 4-Phenyl-tetrahydrofurano[2,3-c]pyridine der allgemeinen Formel I, in der $R^2$ die Hydroxygruppe bedeutet, dadurch gekennzeichnet, daß man ein 4,5,6,7-Tetrahydro-4-oxo-furano[2,3-c]pyridin der allgemeinen Formel

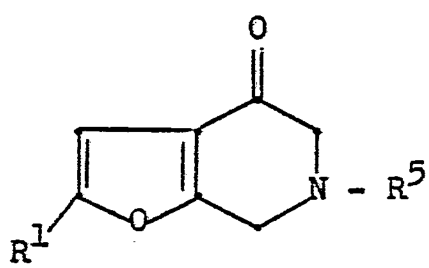

(V)

worin
$R^1$ und $R^5$ die oben angeführte Bedeutung haben, mit einem Phenylmagnesiumbromid der allgemeinen Formel

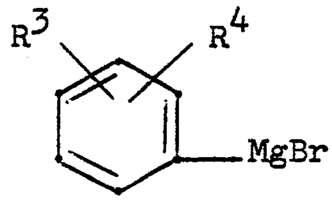

(VI)

worin

$R^3$ und $R^4$ die vorstehend angegebene Bedeutung haben, umsetzt, un daß man gegebenenfalls ein so erhaltenes Endprodukt nach an sich bekannten Methoden in ein physiologisch verträgliches Säureadditionssalz überführt.

12. Verfahren nach Anspruch 11, dadurch gekannzeichnet, daß man zur Abspaltung der 4-Hydroxygruppe eine entsprechende Verbindung der allgemeinen Formel I mit einem Halogenierungsmittel in das Furano-pyridin und dieses mit Methyljodid in das entsprechende Furanopyridiniumjodid umwandelt und letzteres zum entsprechenden Endprodukt mit $R^2$ = Wasserstoff hydriert.

13. Verfahren zur Herstellung neuer 4-Phenyl-tetrahydro-furano[3,2-c]pyridine der allgemeinen Formel II gemäß Anspruch 1, dadurch gekennzeichnet, daß man ein 2-Furfurylamino-1-phenyl-ethanol der allgemeinen Formel

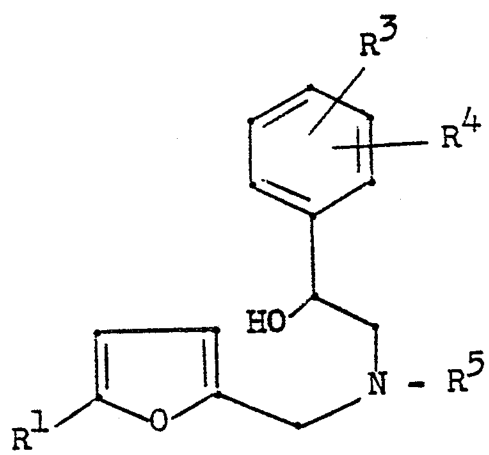

(VII)

worin
die Reste $R^1$, $R^3$, $R^4$ und $R^5$ die oben angeführte Bedeutung haben, mit Trifluoressigsäure oder Trifluorsulfonsäure kondensiert, und daß man gegebenenfalls in an sich bekannter Weise ein so erhaltenes Endprodukt der allgemeinen Formel II in ein physiologisch verträgliches Säureadditionssalz überführt.

14. Verfahren zur Herstellung von Verbindungen gemäß den Formeln I und II in Anspruch 1, dadurch gekennzeichnet, daß man in ein Endprodukt der allgemeinen Formeln I oder II, in denen $R^1$ Wasserstoff bedeutet, nach an sich bekannten Methoden ein Halogenatom, eine Alkylgruppe, eine Dialkylaminogruppe oder eine Pyrrolidinomethylgruppe einführt.

15. Verfahren zur Herstellung von Verbindungen gemäß den Formeln I und II in Anspruch 1, dadurch gekennzeichnet, daß man ein Endprodukt der allgemeinen Formeln I oder II, in denen $R^5$ für den Benzyl- oder den o-Chlorbenzylrest steht, nach an sich bekannten Methoden entbenzyliert.

16. Verfahren zur Herstellung von Verbindungen gemäß den Formeln I und II in Anspruch 1, worin einer oder beide der Reste $R^3$ und $R^4$ eine Methoxygruppe bedeuten, dadurch gekennzeichnet, daß man eine entsprechende Verbindung mit $R^3$ und/oder $R^4$ = Hydroxy in an sich bekannter Weise mit Bortrifluorid/Methanol behandelt.

17. Verfahren zur Herstellung von Verbindungen gemäß den allgemeinen Formeln I und II in Anspruch 1, worin einer oder beide der Reste $R^3$ und $R^4$ eine Acetoxygruppe bedeuten, dadurch gekennzeichnet, daß man eine entsprechende Verbindung mit $R^3$ und/oder $R^4$ = Hydroxy mit Acetanhydrid erhitzt.

18. Pharmzeutische Zusammensetzungen, enthaltend als Wirkstoff eine oder mehrere Verbindungen der allgemeinen Formeln I und II in Kombination mit üblichen Hilfs- und/oder Trägerstoffen.

19. Methode zur Behandlung depressiver Zustände verschiedenster Genese mittels pharmazeutischer Zusammensetzungen gemäß Anspruch 18.